Europäisches Patentamt

European Patent Office

Office européen des brevets

(11) Veröffentlichungsnummer: **0 391 011**
**A1**

## (12) EUROPÄISCHE PATENTANMELDUNG

(21) Anmeldenummer: **89730096.8**

(22) Anmeldetag: **06.04.89**

(51) Int. Cl.⁵: **A61B 19/00**

(43) Veröffentlichungstag der Anmeldung:
**10.10.90 Patentblatt 90/41**

(84) Benannte Vertragsstaaten:
**CH DE ES FR GB IT LI NL**

(71) Anmelder: **Effner GmbH**
**Alt-Lankwitz 102**
**D-1000 Berlin 46(DE)**

(72) Erfinder: **Vaubel, Wolf-Ekkehard, Prof. Dr.**
**Steinäckerstrasse 15**
**D-1000 Berlin 45(DE)**

(74) Vertreter: **Christiansen, Henning, Dipl.-Ing. et al**
**Patentanwalt CHRISTIANSEN Pacelliallee 43/45**
**D-1000 Berlin 33(DE)**

(54) **Hautexpander.**

(57) Hautexpander, bestehend aus einer über einen Schlauch befüllbaren ballonartigen Hülle (1), wobei der Schlauch (3) mit einem Ende mit der Hülle und mit dem anderen Ende mit einem Injektionsdom (5) verbunden ist, wobei zur Schnellbefüllung oder -entlüftung ein weiterer Schlauch (4) mindestens mittelbar mit der Hülle (1) fest verbunden ist.

# Hautexpander

Die Erfindung betrifft einen Hautexpander der im Oberbegriff des Anspruchs 1 angegebenen Art.

Hautexpander werden subcutan implantiert und mit isotonischer Kochsalzlösung aufgefüllt. Sie dienen dazu, Hautbe reiche zu bilden, um verbesserte Abdeckungsmöglichkeiten zu schaffen. Der entstehende Gewebebereich kann als Lappen bei Korrektur oder Rekonstruktion in Zusammenhang mit Defekten dienen oder auch zur Deckung einer implantierten Prothese benutzt werden.

Die Hautexpander bestehen üblicherweise aus einer Hülle aus sehr elastischem Silikonelastomer-Material. Von der Peripherie des Expanders führt ein Verbindungsschlauch zu einem Injektions-Dom. Der Dom besitzt eine im wesentlichen ebene untere Auflagefläche mit einem Widerlager und trägt im oberen kuppelartigen Bereich eine punktierbare Fläche, die mit einer Kanüle geringen Durchmessers durchstoßen werden kann, um den Expander langsam zu füllen und auf diese Weise das darüber liegende Gewebe zu dehnen. Die flache Unterseite des Doms stellt ein Widerlager dar, während die kuppelförmige Fläche im implantierten Zustand durch das Deckgewebe tastbar ist.

Dabei besteht der Nachteil, daß infolge des kleinen Durchmessers der zulässigerweise verwendbaren Injektionsnadel der Auffüllvorgang in der intraoperativen Phase sehr zeitraubend und daher lästig ist.

Des weiteren ist es bekannt, an der flachen Unterseite der Hülle ein Füllventil vorzusehen, welches als sogenanntes Lippenventil ausgeführt ist und den Durchtritt eines weiteren Füllschlauches durch das Ventil hindurch während der Operationsphase zum schnellen Befüllen und Entlüften zuläßt.

Bei diesem System ist nachteilig, daß einerseits ein größerer Herstellungsaufwand entsteht und zum anderen die Behandlung und Bedienung des zusätzlichen Ventils hohe Sorgfalt erfordert, da bei eventuellen Undichtigkeiten Kochsalzlösung innerhalb des Körpers austritt. Ein undichter Expander aber muß entfernt werden und hat daher eine Reoperation zur Folge. Außerdem können mit dem in das Ventil einzuschiebenden Schlauch Verunreinigungen in das Hülleninnere des Expanders gelangen. Der Erfindung liegt die Aufgabe zugrunde, bei einem Gegenstand der vorgenannten Gattung die Möglichkeit einer schnellen intra- und auch postoperativen Befüllung zu schaffen, welche keine wesentliche Erhöhung des Herstellungsaufwands bedeutet und auch hinsichtlich der Behandlung keiner zusätzlichen Sorgfalt und besonderen Behandlung bedarf.

Diese Aufgabe wird durch die im kennzeichnenden Teil des Anspruchs 1 angegebenen Maßnahmen gelöst.

Besonders vorteilhaft bei der Erfindung ist, daß mit dem zweiten Schlauchanschluß eine zusätzliche Befüllungs- bzw. Entleerungsmöglichkeit gegeben ist, die gegebenenfalls auch nach der Operationsphase zur Verfügung steht. Vorteilhaft ist weiterhin, daß der Füllschlauch fest montiert ist und daher nicht bis in das Innere der Hülle durch ein Lippenventil vorgeschoben werden muß, so daß damit das sonst mit dem Einschieben des weiteren Schlauchs möglicherweise verbundene Eindringen von Fremdkörpern oder Körperflüssigkeit in das Innere des Expanders sicher verhindert ist. Auf diese Weise sind insbesondere die mit einer Verunreinigung des Behälterinneren verbundenen nachteiligen Folgen ausgeschlossen.

Gemäß einer vorteilhaften Weiterbildung der Erfindung ist die Befestigung der hüllenseitigen Enden beider Schläuche in einer gemeinsamen Anschlußfläche vorgesehen, die in einem einheitlichen Herstellungsschritt mit der Hülle verbunden werden kann. Der entsprechende flanschartige Ansatz, der den Durchtrittsbereich der Schläuche rundherum umgibt, wird durch Kleben oder durch ein thermisches Verfahren, wie beispielsweise Verschweißen, mit der Hülle verbunden.

Bei einer anderen Ausführungsvariante bildet der weitere Schlauch die Fortsetzung des ersten Schlauches jenseits des Injektionsdomes und ist dort mit dem ersten Schlauch gemeinsam einseitig oder getrennt von diesem an der gegenüberliegenden Seite des Doms befestigt, wobei auch der Vorteil besteht, daß die Befestigung des zweiten Schlauches in einem einheitlichen Fabrikationsschritt erfolgen kann und es daher außer des zusätzlichen Materialaufwands keinerlei den Herstellungsaufwand heraufsetzender Maßnahmen bedarf.

Gemäß einer anderen vorteilhaften Weiterbildung ist der weitere Schlauch an seinem der Hülle abgewandten Ende mit einem Ventil versehen, wie es für Intubationen verwendet wird. Ein derartiges Ventil ist gegenüber den Medien Wasser oder Luft sicher dichtend und läßt sich auf einfache Weise betätigen.

Falls es vorgesehen ist, den Hautexpander unter der Haut zu expandieren, wird der das zusätzliche Ventil enthaltende Schlauch doppelt unterbunden und abgeschnitten, wobei dann das Ventil entfällt und der Restschlauch ohne wesentlichen Raumbedarf an die Hülle angelegt werden kann.

Zusätzlich besteht noch die Möglichkeit, eine Schnellauffüllung über den die Haut perforierenden Schlauch vorzunehmen, wobei das Portventil nach Unterbindung des Schlauchs abgetrennt und der so liegende Katheter des Hautexpanders von außen her mit einem Infusomaten befüllt werden kann. Das Ventil für die zweite Zuleitung kann in diesem Fall für eine spätere Verwendung aufgehoben werden. Je nach Häufigkeit der verschiedenen Anwendungsfälle kann gegebenenfalls dieses Ventil in einer Verkaufspackung dem Hautexpander lose beigelegt werden, so daß es bedarfsweise benutzt oder für eine spätere Anwendung aufgehoben werden kann.

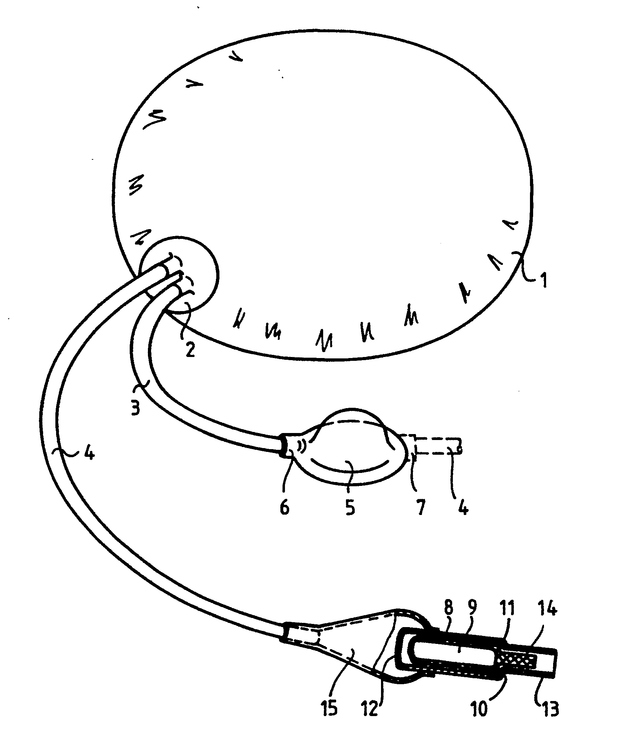

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Zeichnung näher dargestellt. Die einzige Figur zeigt ein Ausführungsbeispiel des erfindungsgemäßen Hautexpanders in perspektivischer Darstellung.

Eine Hülle 1 besteht aus hochelastischem Silikonelastomer und erweitert sich bei Befüllen mit isotonischer Kochsalzlösung ballonartig. Die Hülle 1 ist in den verschiedensten Größen und Formen herstellbar. An der Unterseite im peripheren Randbereich befindet sich eine aus einer flexiblen Materialschicht bestehende flanschartige Anschlußfläche 2, welche den Durchtritt zweier Schläuche 3 und 4 in das Innere der Hülle 1 ermöglicht. Da die Fläche 2 bei der Darstellung in der Figur in der Draufsicht erscheint, ist der Expander in der Ansicht von unten widergegeben. Die im Implantationsfall von der Körperoberfläche abgewandte Fläche befindet sich in dieser Ansicht also oben.

Die Anschlußfläche 2 bildet eine Verstärkung der Hülle 1 und ist an diese flächig angeklebt oder mit dieser durch ein thermisches Verfahren verbunden. Die Fläche 2 bildet damit ein flanschartiges Gebilde, welches aber derart flexibel ist, daß es der Form der Hülle beim Befüllen und auch in ihren sonstigen Bewegungen folgen kann.

Die Silastikschläuche 3 und 4 durchstoßen die Fläche 2 und die Ballonhülle und sind mit der Anschlußfläche 2 ebenfalls flächig verbunden bzw. in mit der Fläche verbundene stutzenförmige Durchlässe eingeklebt. Es ist ersichtlich, daß durch das gemeinsame Verbinden beider Schläuche 3 und 4 mit einer Anschlußfläche 2 insgesamt nur ein die Verbindungen herstellender Arbeitsvorgang erforderlich ist, da beide Schläuche 3 und 4 gleichzeitig in einem Klebe- oder Schweißvorgang angebracht werden können.

Ein Injektionsdom 5 ist in bekannter Weise vorgesehen, wobei die Kuppel des Domes mit einer Injektionsnadel durchstochen werden kann und nach Zurückziehen der Nadel selbstdichtend ist. Dies gilt jedoch nur für eine 23G-Nadel oder kleinere, da beim Durchstechen mit einer größeren Nadel ein Materialzylinder aus der Domkuppel her ausgestanzt würde und die somit entstehende Öffnung durch die Kompressionskraft des Materials nicht wieder dichtend verschlossen werden könnte.

An der Grundfläche des Domes 5 ist ein Widerlager vorgesehen, welches einerseits ein Durchstechen des Bodens mit der Nadel verhindert und zum anderen als Röntgenkennung für das Auffinden des Injektions-Doms dient.

Das von der Hülle 1 abgewandte Ende der ebenfalls aus Silastikmaterial bestehenden Zuleitung 3 ist in einem Stutzen 6 des Doms eingeführt und dort zugfest verbunden.

An dem Injektionsdom 5 ist an der rechen Seite gegenüber dem Stutzen 6 ein weiterer Stutzen 7 gestrichelt angedeutet, der in Verbindung mit einem Schlauch 4′ steht, welcher gegebenenfalls den zweiten Schlauch 4 ersetzt, wobei auch in diesem Fall der Vorteil der einfachen Herstellbarkeit der Verbindungen in einem Arbeitsgang gegeben ist.

Die Enden der Schläuche 3 und 4′ werden in die entsprechenden Stutzen 6 und 7 des Injektionsdomes eingeführt und dort befestigt bzw. werden in eine entsprechende Form eingelegt, so daß die Kuppel des Doms durch Umspritzen der Schlauchenden erzeugt werden kann. In die Form der Domkuppel läßt sich in diesem Fall auch gleichzeitig eine Materialschicht mit hoher innerer Kompressionsfähigkeit einlegen, welches die durchstechbare Schicht bildet, die sich aufgrund ihrer inneren Spannung nach dem Zurückziehen der Injektionsnadel wieder schließt.

Am Ende der zweiten Leitung 4 (bzw. 4′) ist ein Ventil vorgesehen, welches aus einem zylindrischen Behälter 8 aus Kunststoff besteht, welches den Ventilkörper 9 führt. Der Hohlzylinder 8 weist an seinem dem Schlauch 4 abgewandten Ende eine schulterförmige umlaufende dichtende Kante 10 auf, die mit einer entsprechenden Kante 11 des Ventilkörpers 9 in Wechselwirkung steht und den Durchlaß bei Anlage der Schultern 11 des Ventilkörpers schließt. Am gegenüberliegenden Ende des Hohlzylinders 8 ist eine Verengung 12 vorgesehen, welche als Anlagefläche zur Wegbegrenzung des Ventilskörpers 9 in dieser Richtung wirkt, ohne hier jedoch einen dichtenden Abschluß zu bilden. Die hohlzylindrische Hülse setzt sich jenseits der Schulter 10 fort in einem hohlzylindrischen Bereich 13 mit geringerem Querschnitt. Der Ventilkörper 9 besteht aus einem elastischen, gummiartigen Material und ist becherförmig hohl ausgebildet, wobei sich der Boden des Bechers im Bereich der Schulter 11 befindet. In den Bereich des hohlzylindrischen Teils 13 der Hülse 8 setzt sich der Ventilkörper in Form eines Stiels 14 aus Vollmaterial fort, der der Betätigung des Ventils durch Druck auf das Ende des Stiels 14 dient.

Ein elastisches, schlauchartiges Verbindungsstück 15 umschließt einerseits den hohlzylindrischen Körper 8 und andererseits das vom Behälter 1 entfernte Ende der Zuleitung 4. Das Verbindungsstück ist bevorzugt mittels einer thermischen Schrumpfbehandlung aufgebracht, so daß es an beide Elemente hermetisch dichtend anschließt. Das dargestellte Ventil entspricht bauartsmäßig, den bei Intubationen verwendeten Ventilen und ist kostengünstig herstellbar.

Durch die weitere Zuleitung 4 kann jetzt intraoperativ der Behälter 1 schnell befüllt bzw. die darin befindliche Luft entleert werden, so daß die zeitliche Belastung für Arzt und Patient gering ist. Durch die vorteilhafte Möglichkeit, das Ende der Zuleitung 4 auch transcutan zu verlegen, kann weiterhin der Anschluß an eine automatische Infusionseinrichtung erfolgen, welche eine kontinuierliche Befüllung über einen längeren Zeitraum zuläßt, so daß Injektionen nicht oder nur über einen begrenzten zeitlichen Bereich der Behandlung notwendig sind.

Durch die feste hermetische Verbindung der Zuleitungen mit dem Behälter bzw. mit dem Injektionsdom sind sämtliche Anschlüsse vollständig dicht, so daß insbesondere das Eindringen von Partikeln oder auch nur geringen Flüssigkeitsmengen bzw. das Austreten von Kochsalzlösung aus dem Behälter durch ein unbeabsichtigtes Verschieben der weiteren Zuleitung in der Operationsphase mit Sicherheit verhindert ist.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

**Ansprüche**

1. Hautexpander, bestehend aus einer über einen Schlauch befüllbaren ballonartigen Hülle, wobei der Schlauch mit einem Ende mit der Hülle und mit dem anderen Ende mit einem Injektionsdom verbunden ist,
**dadurch gekennzeichnet,**
daß zur Schnellbefüllung oder -entlüftung ein weiterer Schlauch (4) mindestens mittelbar mit der Hülle (1) fest verbunden ist.

2. Hautexpander nach Anspruch 1, **dadurch gekennzeichnet,** daß das freie Ende des weiteren Schlauchs (4) mit einem Ventil (8 bis 15) verbunden ist, dessen absperrbarer Querschnitt größenordnungsmäßig im Bereich des Innenquerschnitts des Schlauchs (4, 4′) liegt.

3. Hautexpander nach Anspruch 2, **dadurch gekennzeichnet,** daß es sich bei dem Ventil um einen rohrartigen Hohlkörper (8) mit darin befindlichem elastischem Körper (9) handelt, der mindestens eine Dichtfläche (11) an der mit einem mit der Hülle (1) verbundenen Bereich abgewandten Seite aufweist, die an einer Gegenfläche (10) des Hohlkörpers (8) anliegt, welche an das nicht mit dem mit der Hülle verbundenen Ende des weiteren Schlauchs (4, 4′) ringförmig anschließt.

4. Hautexpander nach Anspruch 3, **dadurch gekennzeichnet,** daß das Ventil (8 bis 15) einem solchen für Intubationsanwendungen konstruktionsmäßig entspricht.

5. Hautexpander nach einem der vorangehenden Ansprüche, **dadurch gekennzeichnet,** daß die beiden Schläuche (3, 4) in einem gemeinsamen Anschlußflansch enden, der mit dem Ballon verschweißt oder verklebt ist.

6. Hautexpander nach einem der Ansprüche 1 bis 4, **dadurch gekennzeichnet,** daß das behälterseitige Ende des weiteren Schlauchs (4′) mit dem Injektionsdom (5 bis 7) verbunden ist.

EP 0 391 011 A1

Europäisches
Patentamt

# EUROPÄISCHER RECHERCHENBERICHT

Nummer der Anmeldung

EP  89 73 0096

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 183 496  (DOW) <br> * Seite 4, Zeile 29 - Seite 5, Zeile 11; Figur 1 * <br> --- | 1 | A 61 B   19/00 |
| A | US-A-4 643 733  (BECKER) <br> * Spalte 3, Zeile 67 - Spalte 4, Zeile 17; Figur 6 * <br> --- | 1 | |
| A | WO-A-8 706 818  (S+G IMPLANTS) <br> * Anspruch 1; Figur 1 * <br> ----- | 1 | |
| | | | **RECHERCHIERTE SACHGEBIETE (Int. Cl.5)** <br><br> A 61 B <br> A 61 F <br> A 61 M |

Der vorliegende Recherchenbericht wurde für alle Patentansprüche erstellt

| Recherchenort | Abschlußdatum der Recherche | Prüfer |
|---|---|---|
| DEN HAAG | 10-11-1989 | MOERS R.J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

      
& : Mitglied der gleichen Patentfamilie, übereinstimmendes Dokument

EPO FORM 1503 03.82 (P0403)